# DEMANDE DE BREVET EUROPEEN

(11) **EP 4 160 613 A1**
(43) Date de publication de la demande: **05.04.2023**
(21) Numéro de dépôt: 22197399.3
(22) Date de dépôt: 23.09.2022
(51) Int. Cl.: G16H 40/63, A61B 6/00, F16M 11/00, G06F 1/16, A61B 8/00, G06F 3/01

(54) **SYSTÈME DE VISUALISATION MÉDICALE**

(30) Priorité: 29.09.2021 FR 2110274
(71) Demandeur: SUPERSONIC IMAGINE, 13290 Aix-en-Provence (FR)
(72) Inventeur: LERAT, Damien, 13290 Aix-en-Provence (FR)
(74) Mandataire: Paustian & Partner Patentanwälte mbB

(57) **Abrégé**

La présente divulgation concerne un système de visualisation médicale pour visualiser des informations médicales pour un utilisateur du système, configuré pour: recevoir des données représentant les informations médicales, et
recevoir des données représentant une position de l'utilisateur et/ou une direction du regard de l'utilisateur,
le système comprenant :
un dispositif de visualisation qui est configuré pour :
afficher une image basée sur les données représentant les informations, et
ajuster la position et/ou l'orientation de l'image affichée en fonction de la position de l'utilisateur et/ou de la direction du regard.

## Description

### Domaine Technique

La présente invention est relative aux dispositifs d'imagerie médicale. Un tel système peut constituer un système d'examen médical. Plus particulièrement, il peut constituer et/ou être associé à un dispositif à ultrasons.

### Technique antérieure

Un système de visualisation médical comprend habituellement des moyens électroniques, par exemple un capteur et/ou une sonde (par exemple une sonde d'échographie) pour acquérir des données d'un patient, et un processeur pour traiter des données acquises. Il peut comprendre en outre un module de pilotage pour piloter le système, associé notamment à une interface d'utilisateur. Le système médical peut être destiné à fournir des données d'un milieu à examiner, affichées sur un écran. Dans le cas des applications médicales, le milieu est un corps, par exemple une partie du corps d'un patient (muscles, foetus, sein, foie, abdomen, ...). En général, la sonde est maintenue par un opérateur ou un bras robotisé contre la surface d'un milieu observé, afin d'acquérir des données sur ce milieu.

Souvent l'utilisateur du système médical regarde en même temps sur un écran du système. Cependant, l'écran n'est généralement pas directement adjacent au milieu. Par exemple, dans le cas de l'examen d'un patient allongé sur un lit ou assis sur une chaise, l'écran est souvent placé sur un chariot (ou plus généralement une plate-forme) placé à côté du lit ou de la chaise et donc (du point de vue de l'utilisateur) dans une direction complètement différente de celle du milieu. Par conséquent, l'utilisateur du dispositif médical est contraint de prendre une position inconfortable, non ergonomique et non naturelle pendant l'acquisition des données. L'utilisateur est notamment obligé de tourner la tête et généralement de pivoter tout ou partie de son torse.

Dans ce contexte, la littérature internationale montre que 80 à 90 % des échographistes ressentent des douleurs lors de l'exécution d'examens et/ou scans, douleurs qui apparaissent à un moment ou à un autre de leur carrière. Parmi eux, environ 20 % subiront une blessure qui changera leur carrière ou leur vie.

On connaît différents types de système médical. Un système médical, par exemple un dispositif à ultrasons se présente sous la forme susmentionnée d'un chariot à ultrasons, munis généralement de roues (ou plus généralement d'une plate-forme à ultrasons) qui est configuré pour être déplacé sur le sol. Le chariot est configuré pour supporter les parties relativement lourdes du système médical, en particulier son unité de traitement et son ou ses dispositifs d'affichage, souvent de type écran.

De tels système peuvent avoir une qualité d'image de haute qualité et une polyvalence dans les applications de leur utilisation (en particulier grâce à l'utilisation possible de plusieurs sondes), mais ils manquent de maniabilité. Par conséquent, les dispositifs d'écran placés sur le chariot ou fixés à celui-ci ont une mobilité réduite. Les dispositifs d'écran peuvent être rotatifs et, dans certains cas, la position verticale peut être réglable. Cependant, la position relative par rapport à l'utilisateur qui doit se positionner de manière adéquate par rapport au patient reste principalement sensiblement toujours la même. L'utilisateur est donc obligé de tourner/pivoter régulièrement la tête et d'adopter une position inconfortable et non naturelle pour regarder l'écran pendant l'examen d'un patient.

En outre, il existe des dispositifs à ultrasons portables qui utilisent par exemple un smartphone ou une tablette électronique pour l'imagerie. La sonde communique dans ce cas avec le smartphone et/ou la tablette. Ces systèmes peuvent être facilement déplacés. Cependant, ils ne comprennent généralement qu'une seule sonde. En outre, ils ont un écran relativement petit et une autonomie limitée en raison d'une batterie relativement petite. En raison des pièces relativement compactes et légères d'un tel système et de l'autonomie réduite, la qualité de l'image est généralement moins bonne que celle d'un dispositif à ultrasons classique ou a fortiori haut de gamme. En outre, ils doivent toujours être portés à la main, ce qui empêche l'utilisateur d'utiliser ses mains pour d'autres tâches et ce qui peut propager des infections nosocomiales.

Par ailleurs, il existe différentes techniques d'eye-tracking. Par exemple, US2015223684A1 propose d'utiliser un module caméra pour l'eye-tracking. Une rotation d'un écran d'affichage peut être utilisée pour déclencher un changement des caractéristiques opérationnelles du module caméra, en particulier en utilisant un filtre IR interne du module caméra.

### Exposé de l'invention

La présente divulgation a donc pour but de prévoir un système de visualisation médicale pour visualiser des informations médicales qui remédie les inconvénients susmentionnés. En particulier, l'objectif est de fournir un système de visualisation médicale qui permet une visualisation optimisée des informations médicales et un confort d'utilisation. Notamment, il est souhaitable que le système de visualisation médicale permette à l'utilisateur du système d'éviter les positions inconfortables et/ou non naturelles pour considérer informations médicales visualisées pendant l'acquisition des données.

La présente divulgation se rapporte à un système de visualisation médicale pour visualiser des informations médicales par un utilisateur du système.

Le système est configuré pour :
recevoir des données représentant les informations médicales, et
recevoir des données représentant une position de l'utilisateur et/ou une direction du regard de l'utilisateur.

Le système peut comprend un dispositif de visualisation qui est configuré pour :
afficher une image basée sur les données représentant les informations, et
ajuster la position et/ou l'orientation de l'image affichée en fonction de la position de l'utilisateur et/ou de la direction du regard.

En fournissant un tel système, il devient possible de visualiser des informations médicales à une position et/ou une orientation qui convient à l'utilisateur du système, voire qui permet d'optimiser son confort de travail, allant jusqu'à une position considérée naturelle pour l'utilisateur. En particulier, la position et/ou l'orientation des informations peuvent être ajustées de manière à correspondre à la direction du regard de l'utilisateur. En d'autres termes, l'utilisateur n'est pas obligé de tourner la tête pour regarder les informations médicales pendant l'acquisition des données, par exemple pendant l'examen d'un patient, avant l'examen et/ou après l'examen (lorsque l'utilisateur parle au patient par exemple).

L'ajustement peut être fait de manière dynamique de sorte que les informations visualisées suivent la direction du regard de l'utilisateur pendant le mouvement de l'utilisateur (par exemple pendant un changement de position de l'utilisateur en raison d'une intervention ou d'un examen).

Par exemple, la position et/ou l'orientation de l'image affichée peut être ajustée automatiquement. Plus généralement, le système peut être configuré pour détecter un comportement spécifique de l'utilisateur (par exemple un geste et/ou un mouvement et/ou prendre en main un dispositif) et pour s'ajuster en réponse automatiquement. Donc, le système peut permettre une amélioration de l'ergonomie du poste de travail de l'utilisateur. Le système peut également améliorer l'hygiène du poste de travail, car moins de manipulation sont nécessaires, notamment pour manuellement ajuster le dispositif de visualisation. En outre le système peut permettre une réduction du temps d'examen, car il n'y a pas de besoin d'orienter et/ou positionner le dispositif de visualisation avant, pendant et après l'examen.

Les données représentant la position de l'utilisateur peuvent comprendre des données représentant la position du visage et/ou du corps de l'utilisateur. Ces données peuvent permettre de gérer les cas où l'utilisateur tourne le dos au patient (lorsqu'il se déplace par exemple dans la salle de scan, ou dans un bloc d'intervention).

Ces données représentant la position du visage et/ou du corps de l'utilisateur peuvent aussi être utilisées par le système pour authentifier l'utilisateur. Ainsi, la sécurité d'utilisation peut être augmentée, car seulement des personnes autorisées ayant le droit d'utilisation du système peuvent l'utiliser. En cas de tentative d'utilisation par une personne non autorisée, le système peut automatiquement se mettre en veille, ou en défaut de sécurité.

L'ajustement de la position et/ou l'orientation de l'image affichée peut comprendre une rotation/pivotement de l'image affichée et/ou un déplacement translatoire (c'est-à-dire un déplacement du système d'une position dans l'espace à une autre position). En d'autres termes, le mouvement peut être par exemple un mouvement 6D (selon six dimensions) dans l'espace, comprenant un mouvement rotatif tridimensionnel (c'est-à-dire autour des trois axes orthogonaux) et un mouvement rotatif tridimensionnel et un mouvement de translation tridimensionnel (c'est-à-dire un mouvement dans tout ou partie des plans x, y et z).

Les données représentant les informations médicales peuvent comprendre au moins un parmi :
des données acquises relatives à un patient, comme par exemple ses données administratives, personnelles,
des données acquises par un dispositif médical qui est manipulé par un utilisateur du système,
des données acquises antérieurement par un dispositif médical et/ou sauvegardés, et
des données d'un dossier d'un patient.

Le dossier peut comprendre par exemple le dossier médical (comprenant par exemple des données d'examen antérieurs) et/ou administratif du patient. Donc, les informations médicales peuvent comprendre des images, des videos at autres informations visuelles ou sonores, mais aussi du texte, comme le nom du patient etc.

Le dispositif médical peut comprendre au moins l'un des éléments suivants :
une sonde ou autre dispositif d'examen,
un capteur, et/ou
un dispositifs d'intervention avec un capteur.

Le système peut comprendre en outre un capteur optique configuré pour mesurer la position de l'utilisateur et/ou de son visage et/ou la direction du regard de l'utilisateur.

Le capteur optique peut être configuré pour suivre la position de l'utilisateur et/ou du visage et/ou la direction du regard de l'utilisateur.

Le dispositif de visualisation peut être configuré pour ajuster la position et/ou l'orientation de l'image affichée en temps réel ou quasi réel en fonction de la position de l'utilisateur et/ou du visage et/ou de la direction du regard.

Le dispositif de visualisation peut comprendre un système de déplacement comprenant au moins un moteur et/ou un asservissement. Le système de déplacement peut être configuré pour déplacer mécaniquement et/ou physiquement la position et/ou l'orientation du dispositif de visualisation.

Dans cette variante la position et/ou orientation du dispositif de visualisation (par exemple un dispositif d'écran d'affichage) peut être déplacé mécaniquement et/ou physiquement. Donc, le dispositif de visualisation même peut être déplacé.

Le dispositif de visualisation peut comprendre un système optique comprenant au moins un élément optique mobile. Le système optique peut être configuré pour projeter optiquement l'image, tel que la position et/ou l'orientation de l'image affichée soit ajustée.

Donc, le dispositif de visualisation même peut principalement maintenir sa position sans être déplacé. En revanche, l'image affichée peut être déplacée sur le dispositif.

Par exemple, il est possible qu'un tel déplacement soit effectué seulement électroniquement et/ou optiquement. Si le dispositif de visualisation comprend par exemple un écran holographique, il est possible d'orienter et positionner l'hologramme de tel manière que le plan de vue de l'anatomie est ajusté dynamiquement par rapport à l'utilisateur. L'écran holographique peut être par exemple un mur de brume holographique.

L'élément optique mobile peut être configuré pour transmettre une image dans des directions variables. Par exemple, l'élément optique mobile peut pivoter dans ce but. L'élément optique mobile peut comprendre par exemple une lentille optique.

Le dispositif de visualisation peut comprendre un écran d'affichage mobile, optionnellement configuré pour ajuster l'orientation de l'image affichée en fonction de la position de l'utilisateur et/ou de son visage.

Cet écran d'affichage mobile peut être par exemple un dispositif avec un écran d'affichage, qui peut se pivoter et/ou déplacer selon un mouvement de translation, notamment en fonction de la position de l'utilisateur et/ou de la direction du regard de l'utilisateur. L'écran d'affichage peut donc être mobile.

L'écran d'affichage mobile peut être un écran tactile et/ou peut comprendre un panneau de commande configuré pour permettre à l'utilisateur ou à un ses assistants ou collègues de commander le système.

Le dispositif de visualisation peut comprendre un projecteur d'images configuré pour projeter l'image pour l'afficher par exemple sur une surface et/ou l'afficher comme hologramme (par exemple dans un espace et/ou sur un mur de brume), et optionnellement ajuster la position de l'image affichée en fonction de la direction du regard.

Le projecteur d'images peut être configuré pour projeter l'image face à l'utilisateur (par exemple sur une surface face à l'utilisateur et/ou l'afficher comme hologramme) et/ou en surimpression sur une surface d'une région du milieu, par exemple celle dont les données sont acquises.

Il est cependant possible que le système comprenne et/ou collabore avec un casque (casque de réalité virtuelle, visiocasque, ....) de l'utilisateur. Par exemple, le dispositif de visualisation peut afficher des informations qui peuvent aussi être visibles pour d'autres personnes. Le visiocasque peut en plus afficher des informations supplémentaires, qui sont seulement visibles pour l'utilisateur qui en est équipé. Par exemple, les informations supplémentaires peuvent se superposer et enrichir et/ou compléter les informations affichées par le dispositif de visualisation.

Les informations supplémentaires peuvent contenir par exemple des informations techniques et/ou des informations confidentielles telles que des parties du dossier médical du patient, et/ou sensibles (par exemple, concernant une maladie détectée basé sur les données acquises) qui ne sont pas destinées au patient ou à une autre personne éventuellement présente.

Le système peut comprendre un premier mode, dans lequel le dispositif de visualisation peut être configuré pour ajuster la position et/ou l'orientation de l'image affichée de sorte que l'image suive automatiquement la position de l'utilisateur et/ou du visage et/ou du regard de l'utilisateur.

Le système peut comprendre un deuxième mode, dans lequel le dispositif de visualisation peut être configuré pour afficher l'image à une position fixe et/ou réglable par l'utilisateur.

Le système peut être configuré pour recevoir des informations d'état représentant l'état d'opération du dispositif médical.

Les informations d'état peuvent comprendre :
des informations déterminés en fonction que le dispositif médical est placé dans un porte-dispositif ou non, et/ou
des informations déterminés en fonction de au moins l'un des éléments suivants:
   un temps d'absence de mouvement du dispositif médical (durant une période prédéfinie et/ou paramétrable),
   un état d'acquisition de données par le dispositif médical, et
   un état d'opération selon un protocole d'examen et/ou d'intervention prédéfini.

Le premier mode peut être activé et/ou maintenu en fonction des informations d'état.

Le capteur optique peut être configuré pour détecter au moins un geste prédéfini de l'utilisateur.

Le dispositif de visualisation peut être configuré pour :
déplacer l'image affichée en fonction d'un premier geste prédéfini, et/ou
arrêter ou activer le premier mode en fonction d'un deuxième geste prédéfini, et/ou
ajuster le contenu et/ou la taille de l'image en fonction d'un troisième geste prédéfini.

Selon un autre exemple, si l'utilisateur s'approche de l'image affichée, l'image peut être agrandie automatiquement. Ce geste peut en effet être interprété de telle sorte que l'utilisateur souhaite voir l'image plus en détail.

Selon un autre exemple, si l'utilisateur se déplace dans la pièce, l'acquisition des données par la sonde peut être arrêtée automatiquement. En effet, ce comportement de l'utilisateur peut être interprété comme signifiant que l'utilisateur n'acquiert plus de données mais qu'il change de lieu ou de patient ou autre modification dans la ou les sessions d'examen. La consommation inutile d'énergie peut ainsi être automatiquement réduite. En outre, la coupure de l'acquisition prévient l'usure prématurée des éléments électromécaniques de la sonde ainsi que la chauffe engendrée par la diminution de la dissipation thermique liée à la perte de contact avec le milieu scanné .

Selon un autre exemple, le système peut être configuré pour détecter les accessoires (et/ou dispositifs médicaux) utilisés par l'utilisateur et réagir en fonction de ceux-ci. Par exemple, si l'utilisateur saisit (ou prend en main) un dispositif médical spécifique (par exemple, une sonde ou une aiguille de biopsie), le système peut automatiquement lancer un mode spécifique.

Donc, le système peut généralement être configuré pour détecter un comportement spécifique de l'utilisateur et pour s'ajuster en réponse automatiquement.

Le dispositif de visualisation peut aussi être configuré pour être contrôlable par l'utilisateur par des moyens différents que des gestes visuels. Par exemple, le système peut aussi être configuré pour un control par la voix et/ou par une interface d'utilisateur physique (par exemple une console de control, et/ou des boutons par exemple situés sur la sonde et/ou sur la tablette ou console).

Le système de visualisation médicale peut comprendre en outre au moins l'un des éléments suivants :
un dispositif médical,
une unité de traitement configurée pour traiter les données acquises par le dispositif médical et pour sortir les données traitées au dispositif de visualisation,
un deuxième écran d'affichage configuré pour afficher au moins une partie de l'image affichée par le dispositif de visualisation et/ou une interface utilisateur configurée pour permettre à l'utilisateur de contrôler le système,
un dispositif de montage, sur lequel est monté le dispositif de visualisation et optionnellement l'unité de traitement et/ou le deuxième écran d'affichage.

Les caractéristiques et avantages de l'invention apparaitront à la lecture de la description, donnée uniquement à titre d'exemple non limitatif, et faite en référence aux figures annexées. Notamment, les exemples illustrés dans les figures peuvent être combinés sauf incohérence notoire.

### Brève description des figures

[Fig. 1a] montre schématiquement un premier mode de réalisation donné à titre d'exemple d'un système de visualisation médicale selon la présente divulgation, selon une vue latérale.
[Fig. 1b] montre schématiquement le système de visualisation médicale de la figure 1a avec des modifications optionnelles et avec un ajustement de la position et/ou l'orientation du dispositif de visualisation selon un première exemple.
[Fig. 1c] montre schématiquement le système de visualisation médicale de la figure 1a avec un ajustement de la position et/ou l'orientation du dispositif de visualisation selon un deuxième exemple.
[Fig. 1d] montre schématiquement le système de visualisation médicale de la figure 1a avec un ajustement de la position et/ou l'orientation du dispositif de visualisation selon un troisième exemple.
[Fig. 2a] montre schématiquement un deuxième mode de réalisation donné à titre d'exemple d'un système de visualisation médicale selon la présente divulgation dans une vue en perspective.
[Fig. 2b] montre schématiquement le système de visualisation médicale de la figure 2a dans une vue en perspective du système vu depuis le haut.
[Fig. 2c] montre schématiquement le système de visualisation médicale de la figure 2b avec un ajustement de la position et/ou l'orientation du dispositif de visualisation selon un quatrième exemple.
[Fig. 3a] montre schématiquement un troisième mode de réalisation donné à titre d'exemple d'un système de visualisation médicale selon la présente divulgation dans une vue en perspective.
[Fig. 3b] montre schématiquement le système de visualisation médicale de la figure 3a dans une vue en perspective du système vu depuis le haut et avec une image projetée.

### Description des modes de réalisation

Sur les différentes figures, fournies à titre d'illustration, les mêmes références numériques désignent des éléments identiques ou similaires. Les différents modes de réalisation présentés dans les figures peuvent être combinés sauf incohérence notoire.

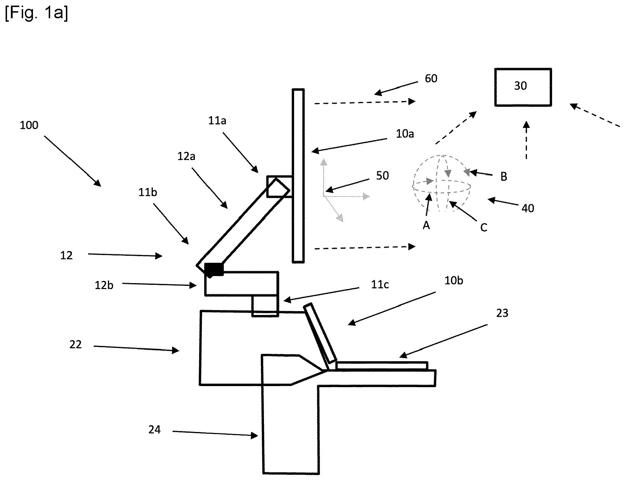

La figure 1a montre schématiquement un premier mode de réalisation donné à titre d'exemple d'un système de visualisation médicale selon la présente divulgation, selon une vue latérale.

Le système de visualisation médicale 100 peut être configuré pour visualiser des informations médicales pour un utilisateur du système.

Le système 100 peut être un système d'imagerie médicale (par exemple pour un examen d'un patient et/ou à usage vétérinaire ou pour toute autre application d'imagerie), en particulier un système à ultrasons. D'autres exemples de tels systèmes comprennent un système d'imagerie optique, un système de radiographie, de la tomographie par ordinateur, un système de mammographie et autres.

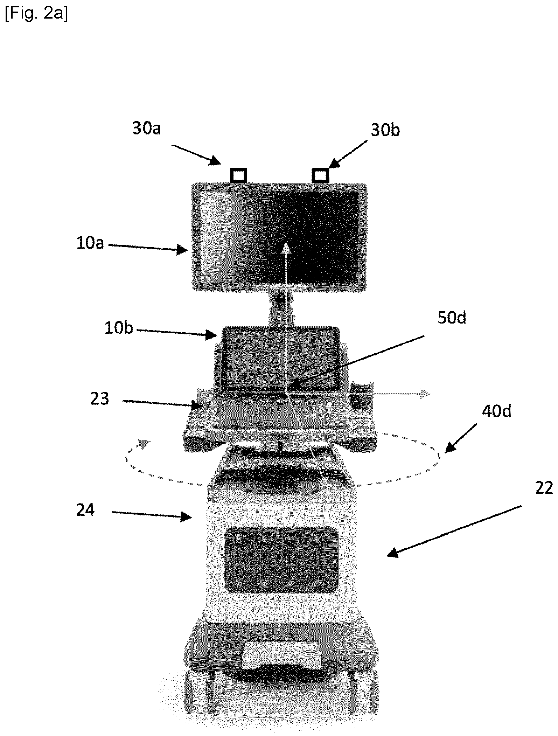

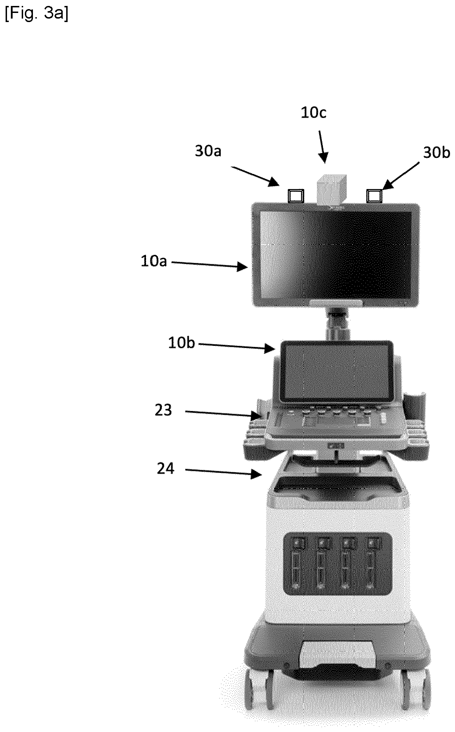

Le système est configuré pour recevoir des données représentant les informations médicales (par exemple acquises à l'aide d'une sonde, non-illustrée dans la figure 1a), et pour recevoir des données représentant une position de l'utilisateur et/ou une direction du regard de l'utilisateur (par exemple par le capteur 30 dans la fig. 1a ou les capteurs 30a, 30b dans la fig. 2a). Le système comprend au moins un dispositif de visualisation 10a, 10b (cf. par exemple fig. 1a), et/ou 10c (cf. par exemple fig. 3a) qui est configuré pour afficher une image basée sur les données représentant les informations, et ajuster la position et/ou l'orientation de l'image affichée en fonction de la position de l'utilisateur et/ou de la direction du regard.

Le système 100 peut en outre comprendre une station principale 22 comprenant par exemple des moyens électroniques et/ou informatiques. Les exemples illustrés ici incluent une unité de traitement et/ou une ou plusieurs zone mémoire. Lesdits moyens informatiques peuvent être utilisés pour traiter les données d'examen. Il est cependant également possible que ces moyens soient déportés (par exemple sur une station de travail, dans le cloud, ....).

La station principale 22 peut être connectée à ou peut comprendre un ou plusieurs capteurs et/ou sondes (non illustrés sur la figure 1a). Lesdits capteurs et/ou sondes peuvent être configurés pour acquérir des données d'examen d'une personne examinée et/ou pratiquer des interventions.

Le dispositif de visualisation peut communiquer avec la station principale 22 via un câble, mais également de manière alternative via une interface sans fil, par exemple en utilisant des protocoles tels que WIFI^{®}, BLUETOOTH^{®} ou autre. La station principale peut également alimenter un dispositif à écran tactile en énergie électrique, ce qui peut également être réalisé alternativement sans fil ou via un connecteur.

La station principale peut être placée sur un pied ou une base 24 (pas entièrement représenté sur la figure 1a). Le pied 24 peut aussi comprendre la station principale 22 (cf. par exemple la fig. 2a). En particulier, le dispositif de montage ou pied 24 peut avoir une hauteur adaptée ou adaptable à la taille d'un utilisateur, c'est-à-dire telle que le dispositif de visualisation 10a se trouve à un niveau approprié pour l'utilisateur et/ou adaptable au type d'examen pratiqué.

Le système, notamment le dispositif de visualisation, peut comprendre un premier écran d'affichage 10a.

Le système, notamment le dispositif de visualisation, peut en outre comprendre un deuxième écran d'affichage 10b. Au moins un des premiers et deuxièmes écrans d'affichage 10a, 10b peut être un écran tactile.

Le système peut en outre comprendre une interface utilisateur et/ou un panneau de commande 23 configuré pour permettre à l'utilisateur de commander le système. Le panneau de commande peut par exemple comprendre un ou plusieurs boutons, des contrôleurs coulissants, et/ou un pavé tactile.

Comme déjà mentionné, le système peut comprendre et/ou peut être configuré pour collaborer avec un dispositif médical (non représenté sur la figure 1a), par exemple une sonde ou un autre dispositif d'examen, un capteur, et/ou un dispositif d'intervention avec un capteur.

La sonde peut être par exemple une sonde à ultrasons. Toutefois, la sonde peut également être configurée pour utiliser d'autres technologies que la technique d'acquisition par ultrasons. Par exemple, elle peut être ou comprendre d'autres types de capteurs ou de techniques d'acquisition de données combinées. Dans un exemple, la sonde peut comprendre un ou plusieurs capteurs et/ou transducteurs optiques et/ou lasers. Il est également possible que la sonde soit configurée pour une acquisition de données opto-acoustique. En conséquence, l'unité de traitement peut être configurée pour traiter également d'autres types de données que les données ultrasonores, afin de générer des informations à des fins d'affichage. Par ailleurs, outre la sonde, le système peut également être configuré pour collaborer avec d'autres types de dispositifs médicaux, par exemple un un marqueur et/ou un ou plusieurs dispositifs d'intervention (par exemple un dispositif configuré pour retirer de la matière du milieu, comme par exemple une aiguille de biopsie ou tout instrument chirurgical utilisé en corrélation avec le dispositif)). En conséquence, le système 100 peut être configuré pour comprendre ou être utilisé avec toute combinaison de ces sondes, capteurs et dispositifs médicaux.

Le système peut en outre comprendre ou être configuré pour collaborer avec un capteur optique 30 configuré pour localiser et/ou mesurer la position de l'utilisateur et/ou du visage de l'utilisateur et/ou la direction du regard de l'utilisateur. Toutefois, comme le montre l'exemple de la figure 1a, le capteur peut également être un dispositif externe par rapport au système 100. Par exemple, le capteur optique 30 peut être disposé dans une pièce ou un environnement où le système est utilisé, de sorte que le capteur 30 puisse détecter la position de l'utilisateur et/ou la direction du regard de l'utilisateur.

De plus, au moins un des premier et deuxième écrans d'affichage 10a, 10b peut être configuré de telle sorte que sa position et/ou son orientation soit automatiquement ajustée en réponse à la position de l'utilisateur ou à la direction du regard de l'utilisateur.

En particulier, l'ajustement peut comprendre un mouvement transversal dans au moins une direction d'axe dans un système de coordonnées cartésiennes tridimensionnel 50 (c'est-à-dire dans au moins une des directions x, y, z) et/ou un mouvement de rotation dans au moins une direction de rotation dans un système de coordonnées polaires tridimensionnel 40 (c'est-à-dire dans au moins un des axes A, B, C). En conséquence, le mouvement peut comprendre des composantes de mouvement entre 1D (unidimensionnel) et 6D (six dimensions). Un mouvement 6D (six dimensions) peut comprendre un mouvement de rotation tridimensionnel et un mouvement de translation tridimensionnel (c'est-à-dire un mouvement dans les plans x, y et z).

Dans l'exemple illustré à la figure 1a, au moins la position et/ou l'orientation du premier écran d'affichage 10a est réglable. La position représentée sur la figure 1a peut également être désignée comme une position par défaut et/ou de parking du premier écran d'affichage 10a. Toutefois, des éléments et/ou fonctions de réglage (c'est-à-dire d'ajustement) correspondants peuvent également être présents pour le deuxième écran d'affichage 10b ou tout autre écran.

Le premier écran d'affichage 10a peut comprendre un système de déplacement comprenant au moins un moteur et/ou un servomoteur. Le système de déplacement peut être configuré pour déplacer mécaniquement et/ou physiquement la position et/ou l'orientation du premier écran d'affichage 10a. Ainsi, le premier écran d'affichage 10a lui-même peut être déplacé.

En particulier, le premier écran d'affichage 10a peut être monté sur un dispositif de support mobile 12. Le dispositif de support mobile 12 peut comprendre au moins un bras 12a, 12b. En outre, le dispositif de support mobile 12 peut comprendre au moins une charnière pivotante (ou joint) 11a, 11b, 11c. Cette charnière pivotante 11a, 11b, 11c peut comprendre une charnière 11a entre un bras 12a et l'écran d'affichage, une charnière 11b entre un premier bras 12a et un deuxième bras 12b, et/ou une charnière 11c entre un bras 12b et le dispositif de montage 24 ou tout autre élément du système (par exemple la station principale 22). La ou les charnières peuvent être rotatives autour d'un ou plusieurs axes. Au moins un des bras peut être extensible et rétractable, de sorte que sa longueur peut être ajustée en fonction de l'utilisation et du mode. Le mouvement de pivotement des charnières et le mouvement de translation d'un bras extensible peuvent être actionnés par un moteur et/ou servomoteur.

En conséquence, grâce à ces mouvements de pivotement et de translation, le premier écran d'affichage 10a peut être déplacé en translation et/ou en rotation. Le dispositif de support mobile 12 peut par exemple être configuré pour permettre une orientation quelconque du premier écran d'affichage 10a. Plus particulièrement, le dispositif de support mobile 12 peut permettre une rotation de l'écran d'affichage 10a (vu depuis la direction d'affichage 60) autour d'un premier axe A (c'est-à-dire un axe vertical) et/ou d'un deuxième axe B (c'est-à-dire un axe horizontal), les deux axes étant orthogonaux. En outre, le dispositif de support mobile 12 peut par exemple être configuré pour permettre tout mouvement de translation dans la plage possible du dispositif de support mobile 12 entièrement déployé.

Le premier écran d'affichage 10a peut être configuré pour ajuster la position et/ou l'orientation en temps réel ou quasi réel en fonction de la position et/ou du visage et/ou de la direction du regard de l'utilisateur. Par exemple, le premier écran d'affichage 10a peut être configuré pour ajuster la position et/ou l'orientation de sorte que l'écran suive automatiquement la position de l'utilisateur et/ou le visage et/ou le regard de l'utilisateur. En conséquence, l'utilisateur peut se déplacer ou changer de position (par exemple de la position debout à la position assise) sans devoir ajuster la position de l'écran tout en pouvant continuer à consulter les informations affichées. La direction principale d'affichage 60 du premier écran d'affichage 10a peut donc suivre la position et/ou la direction du regard de l'utilisateur, de sorte que l'utilisateur peut avantageusement voir les informations affichées. Même si un mouvement de translation du premier écran d'affichage 10a peut être limité en raison de la longueur maximale du dispositif de support mobile 12 entièrement déployé, le premier écran d'affichage 10a peut être tourné autour du premier axe A (c'est-à-dire un axe vertical) et/ou du deuxième axe B (c'est-à-dire un axe horizontal dans le plan d'affichage) pour suivre la position de l'utilisateur et/ou la position de la tête et/ou du visage de l'utilisateur.

Ledit ajustement automatique peut être effectué par le système au moins dans un premier mode.

Le système peut également comprendre un deuxième mode, dans lequel le premier écran d'affichage 10a peut être configuré pour rester dans une position fixe et/ou réglable par l'utilisateur. Ainsi, l'utilisateur peut ne pas être distrait par un mouvement du premier écran d'affichage 10a, lorsque le premier mode n'est pas souhaité par l'utilisateur.

Par exemple, l'utilisateur peut activer et/ou désactiver le premier mode manuellement, par une commande vocale ou tout autre système d'interface homme machine. Alternativement, ou en plus, le premier mode peut être activé et/ou maintenu sur la base des informations d'état représentant l'état de fonctionnement du dispositif médical. Les informations d'état peuvent comprendre par exemple des informations déterminées en fonction du fait que le dispositif médical est placé dans un dispositif de montage, et/ou des informations déterminées en fonction d'au moins l'un des éléments suivants : un temps d'absence de mouvement du dispositif médical (sur une période prédéfinie et/ou configurable), et/ou un état d'acquisition de données par le dispositif médical, et/ou un état de fonctionnement selon un protocole d'examen et/ou d'intervention prédéfini.

En outre, le capteur optique 30 peut détecter au moins un geste prédéfini de l'utilisateur. En réponse, le premier écran d'affichage 10a peut se déplacer en fonction d'un premier geste prédéfini détecté, et/ou arrêter ou activer le premier mode en fonction d'un deuxième geste prédéfini détecté, et/ou ajuster le contenu et/ou la taille d'une image affichée en fonction d'un troisième geste prédéfini du premier écran d'affichage 10a.

Le mouvement du premier écran d'affichage 10a peut également être contrôlé par l'utilisateur au moyen d'autres gestes que les gestes visuels. Par exemple, le système peut également être configuré pour être commandé par la voix et/ou par une interface utilisateur physique 23, une télécommande, etc.

En outre, le mouvement peut être contrôlé par des capteurs de différents types (par exemple le capteur optique 30 et/ou un ou plusieurs capteurs de distance fixés au premier écran d'affichage 10a), qui reconnaissent un objet dans l'environnement du système (par exemple un lit, un siège ou un patient, les murs ou le plafond de la pièce). En conséquence, le mouvement peut être limité de manière à empêcher le premier écran d'affichage 10a de toucher de tels objets (par exemple un patient).

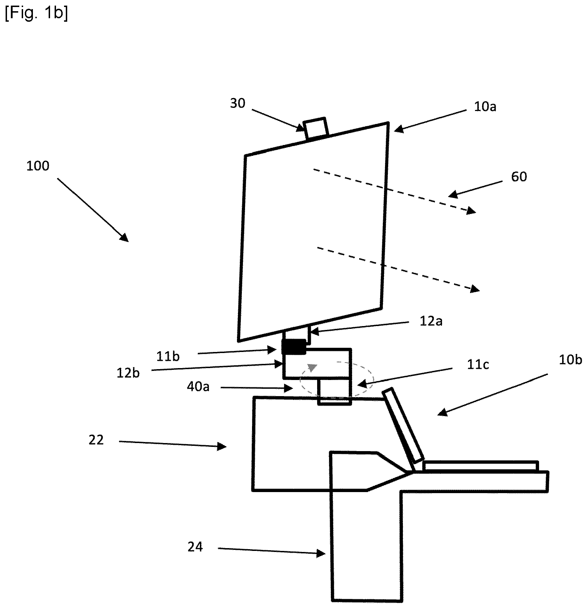

La figure 1b représente schématiquement le système de visualisation médical de la figure 1a avec des modifications optionnelles et avec une position et/ou une orientation ajustée du dispositif de visualisation selon un premier exemple.

Le mode de réalisation selon l'exemple représenté sur la figure 1b peut correspondre à celui de la figure 1a. En particulier, le mode de réalisation selon l'exemple de la figure 1b peut comprendre les mêmes éléments et/ou fonctions.

Toutefois, dans l'exemple illustré, le capteur 30 peut être fixé au système 100 et/ou en faire partie. Par exemple, le capteur peut être fixé sur le premier écran d'affichage 10a, par exemple sur sa partie supérieure.

Dans l'exemple de la figure 1b, le dispositif de support mobile 12 peut être configuré pour permettre une rotation du premier écran d'affichage 10a (vu depuis la direction d'affichage 60) au moins (ou uniquement) autour d'un axe vertical. Cet exemple peut constituer un mode de réalisation élémentaire de la présente divulgation. Dans la position d'ajustement illustrée du premier écran d'affichage 10a (par rapport à la position de l'exemple de la figure 1a), le premier écran d'affichage 10a a été tourné dans le sens des aiguilles d'une montre 40a, vu du dessus. En particulier, le premier écran d'affichage 10a peut être tourné autour de la charnière 11c. Cependant, il peut également tourner autour de toute autre charnière, par exemple la charnière 11b ou la charnière 11a.

Le premier écran d'affichage 10a peut être librement rotatif sur 360°. En variante, le premier écran d'affichage 10a peut pivoter de 180° (ou moins) dans les deux sens de rotation à partir de la position par défaut représentée sur la figure 1a. Cette alternative peut être utilisée, par exemple, lorsque le premier écran d'affichage 10a est relié par un câble à la station principale 22 qui ne permet pas une ou plusieurs rotations de 360°.

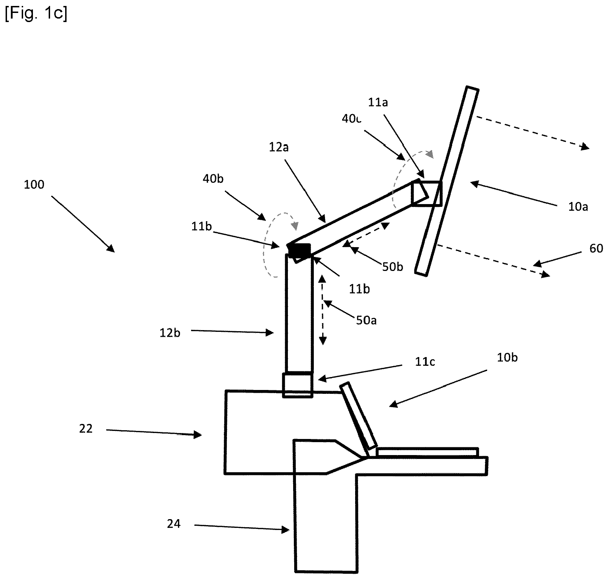

La figure 1c représente schématiquement le système de visualisation médicale de la figure 1a avec une position et/ou une orientation ajustée du dispositif d'affichage selon un deuxième exemple.

Le mode de réalisation selon l'exemple représenté sur la figure 1c peut correspondre à celui de la figure 1a ou 1b. En particulier, le mode de réalisation selon l'exemple de la figure 1c peut comprendre les mêmes éléments et/ou fonctions. En particulier, le mode de réalisation selon l'exemple de la figure 1c peut comprendre les mêmes fonctions de rotation que celui de la figure 1b.

En outre, les bras 12a et 12b peuvent être extensibles et rétractables. Dans la position d'ajustement illustrée du premier écran d'affichage 10a (par rapport à la position de l'exemple de la figure 1a), les deux bras 12a, 12b peuvent être étendus de sorte que le premier écran d'affichage 10a peut être déplacé en translation vers une position étendue, dans l'exemple illustré vers une position soulevée et faisant saillie horizontalement. En outre, les charnières 11b et 11c peuvent être tournées, de sorte que les bras 12a et 12b se déplient. En outre, la charnière 11a peut être tournée, de sorte que le premier écran d'affichage 10a est légèrement incliné vers le bas.

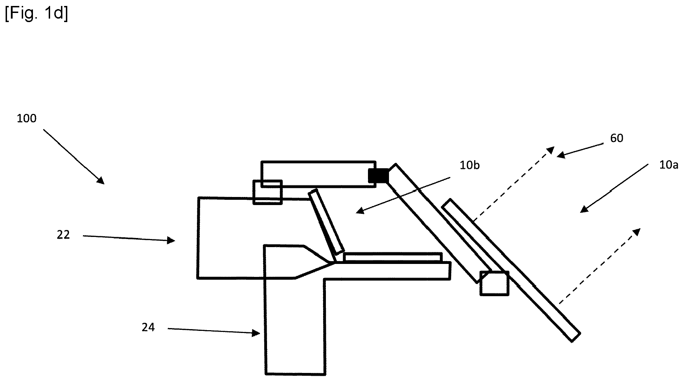

La figure 1d représente schématiquement le système de visualisation médical de la figure 1a avec une position et/ou une orientation ajustée du dispositif d'affichage selon un troisième exemple.

Le mode de réalisation selon l'exemple représenté sur la figure 1d peut correspondre à celui des figures 1a, 1b ou 1c. En particulier, le mode de réalisation selon l'exemple de la figure 1c peut comprendre les mêmes éléments et/ou fonctions. Plus particulièrement, le mode de réalisation selon l'exemple de la figure 1d peut comprendre les mêmes fonctions de rotation et d'extension que celui de la figure 1c. En outre, dans la position d'ajustement illustrée du premier écran d'affichage 10a, les bras 12a, 12b peuvent être étendus de la même manière que sur la figure 1c. Toutefois, par rapport à la position de l'exemple de la figure 1c, les charnières 11b et 11c peuvent être tournées, de sorte que les bras 12a et 12b s'étendent vers une position inférieure qui peut être encore plus en saillie par rapport au dispositif de montage 24 dans une direction horizontale (par exemple vers un utilisateur situé à droite du système sur la figure 1d). En outre, la charnière 11a peut être tournée, de sorte que le premier écran d'affichage 10a est légèrement incliné vers le haut.

L'ajustement illustré de la position et de l'orientation du premier écran d'affichage 10a peut suivre la direction du regard de l'utilisateur. Par exemple, l'utilisateur peut avoir une position légèrement éloignée par rapport au système, par exemple en raison d'un patient positionné entre l'utilisateur et le système. Le patient peut être positionné par exemple sur un lit ou un siège.

Par exemple, dans la position de la figure 1c, l'utilisateur peut lancer une procédure d'examen. En conséquence, l'utilisateur peut regarder légèrement vers le haut, vers un point situé au-dessus du patient. Par exemple, l'utilisateur peut faire une sélection dans un menu affiché sur le premier écran d'affichage 10a, par exemple via un geste ou un autre type de commande, comme décrit ci-dessus. Ensuite, dans la position de la figure 1d, l'utilisateur peut acquérir des données à partir d'un milieu du patient en utilisant une sonde. En conséquence, l'utilisateur peut regarder légèrement vers le bas en direction d'un point de la surface du patient, où se trouve la sonde. Par conséquent, le premier écran d'affichage 10a peut être déplacé de la position et de l'orientation de la figure 1c à celles de la figure 1d. Par conséquent, l'utilisateur peut surveiller toute information fournie sur le premier écran d'affichage 10a dans le contexte de l'acquisition de données. En même temps, la direction du regard de l'utilisateur peut rester sensiblement focalisée sur le point milieu étudié, sur la partie du corps du patient par exemple.

La figure 2a représente schématiquement un deuxième exemple de réalisation d'un système de visualisation médical selon la présente divulgation dans une vue en perspective. Le mode de réalisation selon l'exemple représenté sur la figure 2a peut correspondre à l'une quelconque des figures précédentes. En particulier, le mode de réalisation selon l'exemple de la figure 2a peut comprendre les mêmes éléments et/ou fonctions.

Toutefois, dans l'exemple illustré, le dispositif de montage 24 peut être configuré de telle sorte qu'une extrémité supérieure du dispositif de montage 24 est rotative autour d'un axe vertical, par exemple dans une direction 40d et/ou dans sa direction opposée. Optionnellement, le pied ou base 24 peut comprendre la station principale 22. En conséquence, la combinaison des parties supérieures du système 100 (comprenant par exemple : la station principale 22 (en option, si elle n'est pas située dans le pied), l'interface utilisateur 23 et les premier et deuxième écrans d'affichage 10a, 10b) peut être rotative. Dans une autre option, le dispositif de montage 24 peut être configuré pour permettre un mouvement de translation de ces parties supérieures dans au moins une direction d'axe d'un système de coordonnées cartésiennes tridimensionnel 50d.

Par ailleurs, comme illustré dans l'exemple de la figure 2a, le système peut comprendre au moins deux capteurs optiques 30a, 30b, par exemple fixés sur le premier écran d'affichage 10a, pour permettre une meilleure localisation de l'utilisateur dans un espace tridimensionnel. Par ailleurs, les deux capteurs optiques 30a, 30b peuvent présenter principalement les mêmes caractéristiques et/ou fonctions que le capteur optique 30 décrit ci-dessus.

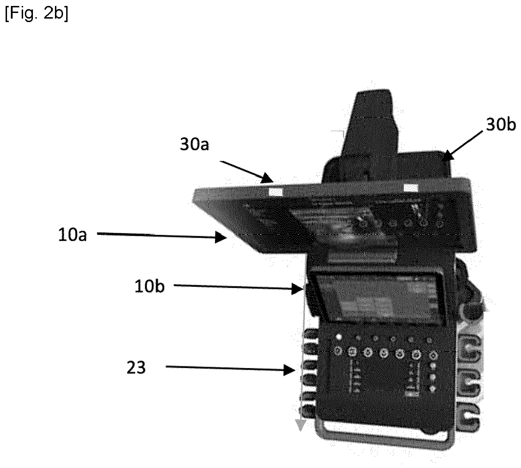

La figure 2b représente schématiquement le système de visualisation médicale de la figure 2a dans une vue en perspective du système depuis le haut. La figure 2b montre schématiquement le système de visualisation médicale de la figure 2a avec une position et/ou une orientation ajustée du dispositif d'affichage selon un quatrième exemple.

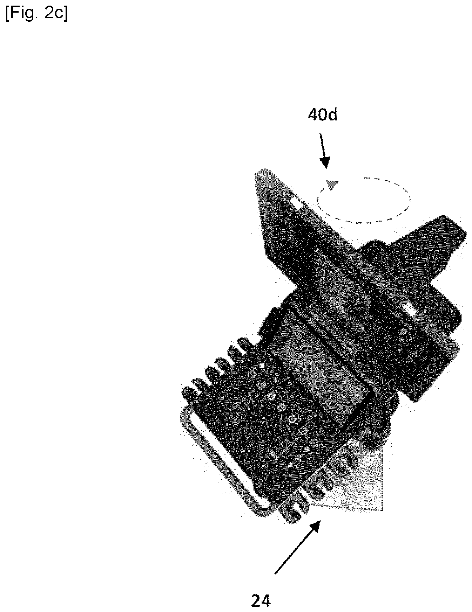

Comme le montre la figure 2c, la combinaison de toutes les parties supérieures du système 100 (comprenant par exemple : la station principale 22 (en option, si elle n'est pas située dans le pied), l'interface utilisateur 23 et le premier et le deuxième écran d'affichage 10a, 10b) peut être orientée par rapport au dispositif de montage 24 dans une direction 40d et/ou dans sa direction opposée. En conséquence, le système peut automatiquement orienter toutes ses parties supérieures vers la position de l'utilisateur. Ainsi, l'utilisateur peut se déplacer autour du système 100 tout en continuant à voir les informations sur le premier écran d'affichage 10a, le deuxième écran d'affichage 10b. En outre, l'utilisateur peut effectuer n'importe quelle commande à l'aide du panneau de commande 23.

La figure 3a représente schématiquement un troisième exemple de réalisation d'un système de visualisation médical selon la présente divulgation dans une vue en perspective. Le mode de réalisation selon l'exemple représenté sur la figure 3a peut correspondre à l'une quelconque des figures précédentes. En particulier, le mode de réalisation selon l'exemple de la figure 3a peut comprendre les mêmes éléments et/ou fonctions.

Toutefois, dans l'exemple illustré, Le système, notamment le dispositif de visualisation, peut comprendre un projecteur d'images 10c.

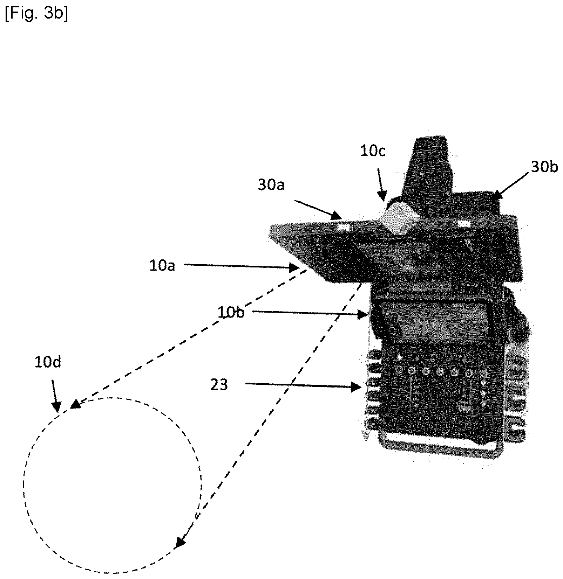

Dans une option, le projecteur d'images 10c peut ajuster mécaniquement sa position et/ou son orientation en fonction de la position et/ou de la direction du regard de l'utilisateur (comme indiqué dans l'exemple des figures 3a et 3b).

En outre ou alternativement, le projecteur d'images 10c peut comprendre un système optique comprenant au moins un élément optique mobile. Le système optique peut être configuré pour contrôler optiquement et/ou électroniquement une direction de projection, de sorte que la position et/ou l'orientation de l'image affichée peut être ajustée, automatiquement ou par l'utilisateur.

Le système 100 peut comprendre une combinaison du projecteur d'images 10c et du premier et/ou du deuxième écran d'affichage 10a, 10b. En variante, le projecteur d'images 10c peut également remplacer ou compléter au moins l'un du premier et du deuxième écrans d'affichages 10a, 10b.

La figure 3b représente schématiquement le système de visualisation médical de la figure 3a dans une vue depuis le haut en perspective du système et avec une image projetée. Le projecteur d'images 10c peut être configuré de telle sorte que la position et/ou l'orientation d'une image projetée 10d peut être ajustée en fonction de la position et/ou de la direction du regard de l'utilisateur et de la position des murs de la pièce. L'ajustement peut suivre les mêmes principes, tels que décrits ci-dessus dans le contexte du premier écran d'affichage 10a. Par exemple, le projecteur d'images 10c peut projeter l'image 10d de manière à ce qu'elle fasse face à l'utilisateur.

Dans un exemple, le projecteur d'images 10c peut projeter l'image 10d sur une surface faisant face à l'utilisateur et/ou la projeter comme un hologramme 3D dans l'espace et/ou comme un hologramme 2D sur un mur virtuel (par exemple un mur de brouillard). En outre, le projecteur d'images 10c peut également projeter l'image 10d de telle sorte qu'elle se superpose à une surface d'une région du support à partir de laquelle les données sont acquises (par exemple sur le patient).

En outre, le système peut détecter la géométrie de la pièce en utilisant les capteurs 30a, 30b, dans laquelle le système est situé. Par exemple, le système peut détecter les lignes formées par les transitions entre des murs et le plafond/sol, et/ou entre des murs pour en déduire l'orientation et la position des murs par rapport au projecteur d'images 10c. Par conséquent, le système peut prendre en compte de telles informations de géométrie, lors de la projection, par exemple pour projeter une image sur un mur de la pièce et/ou projeter un hologramme dans la pièce.

Tous ces modes de réalisations et d'autres exemples tels que décrits ci-dessus sont donnés uniquement à titre d'exemple non limitatif, et peuvent être combinés et/ou modifiés selon la portée des revendications suivantes.

Une référence à un document de brevet ou à tout autre élément identifié comme étant de l'art antérieur ne doit pas être considérée comme une admission que le document ou l'autre élément était connu ou que les informations qu'il contient faisaient partie de la connaissance générale commune à la date de priorité de l'une quelconque des revendications.

## Revendications

1. Un système de visualisation médicale pour visualiser des informations médicales pour un utilisateur du système,
configuré pour :
recevoir des données représentant les informations médicales, et
recevoir des données représentant une position de l'utilisateur et/ou une direction du regard de l'utilisateur,
le système comprenant :
un dispositif de visualisation qui est configuré pour :
afficher une image basée sur les données représentant les informations, et
ajuster la position et/ou l'orientation de l'image affichée en fonction de la position de l'utilisateur et/ou de la direction du regard.

2. Système de visualisation médicale selon la revendication 1, dans lequel les données représentant les informations médicales comprennent au moins un parmi :
des données acquises relatives à un patient,
des données acquises par un dispositif médical qui est manipulé par un utilisateur du système,
des données acquises antérieurement par un dispositif médical et/ou sauvegardés, et
des données d'un dossier d'un patient.

3. Système de visualisation médicale selon la revendication 2, dans lequel le dispositif médical comprend au moins l'un des éléments suivants :
une sonde ou autre dispositif d'examen,
un capteur, et/ou
un dispositifs d'intervention avec un capteur.

4. Système de visualisation médicale selon l'une quelconque des revendications précédentes, dans lequel
le système comprend en outre :
un capteur optique configuré pour mesurer la position de l'utilisateur et/ou la direction du regard de l'utilisateur.

5. Système de visualisation médicale selon la revendication 4, dans lequel le capteur optique est configuré pour suivre la position de l'utilisateur et/ou la direction du regard de l'utilisateur, et/ou
le dispositif de visualisation est configuré pour ajuster la position et/ou l'orientation de l'image affichée en temps réel ou quasi réel en fonction de la position de l'utilisateur et/ou de la direction du regard.

6. Système de visualisation médicale selon l'une quelconque des revendications précédentes, dans lequel
le dispositif de visualisation comprend un système de déplacement comprenant au moins un moteur et/ou un asservissement, le système de déplacement étant configuré pour déplacer mécaniquement la position et/ou l'orientation du dispositif de visualisation.

7. Système de visualisation médicale selon l'une quelconque des revendications précédentes, dans lequel
le dispositif de visualisation comprend un système optique comprenant au moins un élément optique mobile, le système optique étant configuré pour projeter optiquement l'image, tel que la position et/ou l'orientation de l'image affichée soit ajustée.

8. Système de visualisation médicale selon l'une quelconque des revendications précédentes, dans lequel
le dispositif de visualisation comprend un écran d'affichage mobile, optionnellement configuré pour ajuster l'orientation de l'image affichée en fonction de la position de l'utilisateur.

9. Système de visualisation médicale selon l'une quelconque des revendications précédentes, dans lequel
l'écran d'affichage mobile est un écran tactile et/ou comprend un panneau de commande configuré pour permettre à l'utilisateur de commander le système.

10. Système de visualisation médicale selon l'une quelconque des revendications précédentes, dans lequel
le dispositif de visualisation comprend un projecteur d'images configuré pour :
projeter l'image pour l'afficher sur une surface et/ou l'afficher comme hologramme , et
optionnellement ajuster la position de l'image affichée en fonction de la direction du regard.

11. Système de visualisation médicale selon l'une quelconque des revendications précédentes, dans lequel
le projecteur d'images est configuré pour projeter l'image face à l'utilisateur et/ou en surimpression sur une surface d'une région du milieu dont les données sont acquises.

12. Système de visualisation médicale selon l'une quelconque des revendications précédentes, dans lequel
le système comprend un premier mode, dans lequel le dispositif de visualisation est configuré pour ajuster la position et/ou l'orientation de l'image affichée de sorte que l'image suive automatiquement la position de l' utilisateur et/ou du regard de l'utilisateur.

13. Système de visualisation médicale selon l'une quelconque des revendications précédentes, dans lequel
le système comprend un deuxième mode, dans lequel le dispositif de visualisation est configuré pour afficher l'image à une position fixe et/ou réglable par l'utilisateur.

14. Système de visualisation médicale selon l'une quelconque des revendications précédentes, dans lequel
le système est configuré pour recevoir des informations d'état représentant l'état d'opération du dispositif médical.

15. Système de visualisation médicale selon l'une quelconque des revendications précédentes, dans lequel
les informations d'état comprennent :
des informations déterminés en fonction que le dispositif médical est placé dans un porte-dispositif ou non, et/ou
des informations déterminés en fonction de au moins l'un des éléments suivants:
un temps d'absence de mouvement du dispositif médical,
un état d'acquisition de données par le dispositif médical, et
un état d'opération selon un protocole d'examen et/ou d'intervention prédéfini.

16. Système de visualisation médicale selon l'une quelconque des revendications précédentes, dans lequel
le premier mode est activé et/ou maintenu en fonction des informations d'état.

17. Système de visualisation médicale selon l'une quelconque des revendications précédentes, dans lequel
le capteur optique est configuré pour détecter au moins un geste prédéfini de l'utilisateur, et/ou
le dispositif de visualisation est configuré pour :
déplacer l'image affichée en fonction d'un premier geste prédéfini, et/ou
arrêter ou activer le premier mode en fonction d'un deuxième geste prédéfini, et/ou
ajuster le contenu et/ou la taille de l'image en fonction d'un troisième geste prédéfini.

18. Système de visualisation médicale selon l'une quelconque des revendications précédentes, dans lequel
le système de visualisation médicale comprend en outre au moins l'un des éléments suivants :
un dispositif médical,
une unité de traitement configurée pour traiter les données acquises par le dispositif médical et pour sortir les données traitées au dispositif de visualisation,
un deuxième écran d'affichage configuré pour afficher au moins une partie de l'image affichée par le dispositif de visualisation et/ou une interface utilisateur configurée pour permettre à l'utilisateur de contrôler le système,
un dispositif de montage, sur lequel est monté le dispositif de visualisation et optionnellement l'unité de traitement et/ou le deuxième écran d'affichage.
